# EUROPEAN PATENT SPECIFICATION

(11) **EP 2 314 171 B1**
(45) Date of publication and mention of the grant of the patent: **13.11.2019**
(21) Application number: 10183638.5
(22) Date of filing: 13.08.2003
(51) Int. Cl.: A23G 3/00, A23G 3/02, A23G 3/36

(54) **COMPOSITIONS AND PROCESS FOR DELIVERING AN ADDITIVE**
ZUSAMMENSETZUNGEN UND VERABREICHUNGSPROZESS FÜR EINEN ZUSATZSTOFF
COMPOSITIONS ET PROCÉDÉ DE FOURNITURE D'UN ADDITIF

(30) Priority: 13.08.2002 US 403201 P; 16.12.2002 US 433677 P
(43) Date of publication of application: 27.04.2011
(62) Divisional of application: 03785262.1
(73) Proprietor: Intervet International BV, 5831 AN Boxmeer (NL)
(72) Inventor: Huron, Sebastien Claude Jacques, Millsboro, DE DE19966 (US); Cady, Susan, Millsboro, DE DE19966 (US); Pieloch, Mark, Syracuse, NY 68446-0326 (US)
(74) Representative: Intervet International B.V.

(56) References cited:
- EP-A- 0 075 443
- EP-A- 0 273 001
- EP-A- 0 492 235
- EP-A- 1 023 841
- US-A- 5 236 730

## Description

### Background of the Invention

Chewable dosage forms for drug delivery are well known to pharmaceutical technology. It is known in the pharmaceutical industry that the act of chewing increases the surface area of the available active ingredient and may increase the rate of absorption by the digestive tract. Chewable systems are also advantageous where it is desirable to make an active ingredient available topically to the mouth or throat areas for both local effects and/or systemic absorption. Further, chewable dosage forms are also utilized to ease drug administration in pediatric and geriatric patients. Examples of chewable dosage forms may be found in US Pat Nos. 6,387,381; 4,284,652; 4,327,076; 4,935,243; 6,270,790; 6,060,078; 4,609,543; and, 5753,255.

Palatability and "mouth feel" are important characteristics to be considered in providing a dosage form, or matrix, for an active pharmaceutical or medicinal. Unfortunately, many pharmaceuticals and other active ingredients have a bitter or otherwise unpalatable taste, or an unacceptable mouth feel, due to the grittiness or chalkiness of the compound, or both. These characteristics make it difficult to incorporate such active ingredients into the current state of the art for chewable dosage forms because the objectionable taste and/or mouth feel make it less likely to obtain compliance by the user.

As a result, several approaches have been tried in attempting to overcome these problems. The poor taste of a pharmaceutical or other active ingredient may be masked by using suitable flavoring compounds and/or sweeteners. Encapsulation of the active ingredient may also serve to mask bitterness and other undesirable tastes. However, these approaches do not affect the physical state of the dosage form currently employed in the art. For example, chewable vitamin tablets are typically prepared as a compressed, compacted tablet, incorporating one or more active ingredients (e.g., vitamins), a sweetener and flavoring agent to mask the taste of the active ingredients, and a binder, typically microcrystalline cellulose.

Generally, chewable tablets are made by direct compression of a mixture of tableting compounds including the active ingredient, flavorant, binders, etc. The mixture is fed into a die chamber of a tablet press and a tablet is formed by direct compaction. Hardness of the resulting tablet is a direct function of the compression pressure employed. A softer tablet, having an easier bite-through, may be prepared by adding a disintegrant, such as alginic acid, to the pre-tablet mix. Alternatively, a softer tablet may be formed by employing reduced compression pressures. In either case, the resultant tablet is softer, fragile, brittle and easily chipped. Compressed, chewable tablets generally suffer from less than desirable mouth feel, i.e., chalkiness, grittiness, and a dry, powdery taste. Antacid tablets, e.g., Tums.RTM. manufactured by SmithKline Beecham Corp., Pittsburgh, Pa. and Rolaids.RTM. manufactured by Warner Lambert of Morris Plains, N.J., are each examples of typical compressed chewable tablets.

Attempts have been made to reduce the grittiness and/or chalkiness of the compressed tablet by coating particles of the active ingredient with oils or fats, which coat the particles prior to incorporation into the delivery system. In this way, the grittiness or chalkiness of the particles is masked by the oil or fat while the particles are in the mouth. In addition, tablet softness is improved. After swallowing, the oil or fat is removed and the particle can be absorbed by the digestive system. However, the addition of fats or oils to the pre-tablet mix can cause the tableting ingredients to adhere to the die chamber and cause a reduction in the binding action of the binders present in the mix. Accordingly, the art field is in search of a process of manufacturing a soft chew whereby compression and subsequent product loss may be minimized or lessened.

Other techniques for providing a chewable delivery system involve the use of a gum base. Gum bases are insoluble elastomers which form the essential element for chewing gum. The gum base is typically blended with one or more sweeteners to obtain a confectionery gum. A coating containing the active ingredient is then applied over the confectionery gum. As the dosage form is chewed, the coating fractures and/or is dissolved in the mouth and swallowed.

Other delivery systems involve the used of layered, non-homogeneous structures.

Another chewable delivery system is based on a nougat-type, chewy tablet. Such tablets generally employ a base of corn syrup (or a derivative). Such tablets are prepared as a confectionery, i.e., the corn syrup is cooked with water and a binder such as soy protein.

However, the art field has experienced problems with delivering additives/active ingredients to organisms because of palatability issues. Complex guidelines exist along the regulatory framework that make it very difficult to make and/or manufacture a palatable composition with an additive. Accordingly, the art field is in search of a method and/or composition of delivering an additive to an organism in a palatable format.

One part solution is in United States Patent No. 6,387,381 (hereinafter referred to as the '381 patent). The '381 patent discloses an extrudate with formed of a matrix having starch, sugar, fat, polyhydric alcohol and water in suitable ratios such that there exists a water activity of 0.6-0.75, for carrying an active ingredient. The water activity of the product matrix may be adjusted up or down for the active ingredient, be it pharmaceutical, nutraceutical, or a vitamin mineral complex. The claimed product is directed towards a product containing an additive, an extrudate comprising a matrix having about 10 to about 50% wt starch, a sweetener consisting essentially of sucrose, corn syrup and sorbitol, said sucrose being in an amount of at least 10%, and at least about 5% wt water, said composition having A_{w} of about 0.60 to about 0.75, and a soft and chewy texture, and said A_{w} being adjusted to permit an appropriate amount of free water in the presence of the additive. However, this product is limited to an extrudate and not available in a tablet form of formulation.

EP 0492235 discloses edible, non-baked, dried cholestyramine composition with a low moisture content for lowering or controlling blood cholesterol levels.

EP 0075443 discloses a chewable comestible product comprising a frappe component and a syrup component that forms a soft candy such as a nougat, which does not require final forming at elevated temperatures that can be used to deliver an antacid.

US 5,236,730 discloses candy that is formed from a setting material which includes a boiled down, cooled and solidified jelly syrup, mixed and scattered as small pieces in a soft bulk material containing fats and oils which are separately boiled down and cooled.

### SUMMARY OF THE INVENTION

The present invention provides a soft chew, the soft chew comprising:
- a flavoring component of between 0.1 to 50 percent wt,
- a starch component of between 5 to 60 percent wt,
- a sugar component, between 5.0 to 75 percent wt,
- an oil component between 1.0 to 40 percent wt;
- and a pharmaceutical as a first additive,
wherein the moisture content is less than 15 percent wt; wherein the soft chew is formed by knockout, the soft chew is not an extrudate; wherein the composition further comprises an emulsifier component comprising polyethylene glycol as forming agent. In one embodiment soft chew comprises between 5 to 30 percent wt of the emulsifier component.

In one embodiment the soft chew the sugar component act as a sweetener and is selected from white sugar, corn syrup, sorbitol solution, maltitol syrup, oligosaccharide, isomaltooligosacccharide, fructose, lactose, glucose, lycasin, xylitol, lactitol, erythritol, mannitol, isomaltose, polydextrose, raffinose, dextrin, galactose, sucrose, invert sugar, honey, molasses, polyhydric alcohol and mixtures thereof. The the source of the sugar component are corn syrup solids, malt syrup, hydrolyzed corn starch, hydrol, or raw and refined cane or beet sugars.

In one embodiment the soft chew comprises more than one pharmaceutical.

In one embodiment the soft chew comprises polyethylene glycol of different molecular weight, in one embodiment PEG 3350.

In one embodiment the soft chew comprises a meat flavoring component.

Another aspect of the invention is a process for forming a soft chew comprising the steps of:
a) mixing a starch component, a sugar component, an oil component and an emulsifier component comprising polyethylene glycol and a pharmaceutical as first additive component to produce a dough;
b) heating at least a portion of the components;
c) Forming the dough into a soft chew in a forming machine with a knockout such that the soft chew is not an extrudate.

In one embodiment the oil component and the emulsifier component are heated when mixing. In one embodiment in such process the emulsifier component comprises polyethylene glycol, in one embodiment PEG 3350. In one embodiment in step a) the pharmaceutical as first additive is mixed and/ or dissolved in an alcohol or other liquid prior to adding with a dough and/or components of the soft chew.

In one embodiment a soft chew as described above is formed. In onembodiment the step of knocking out is performed on a patty pressing machine.

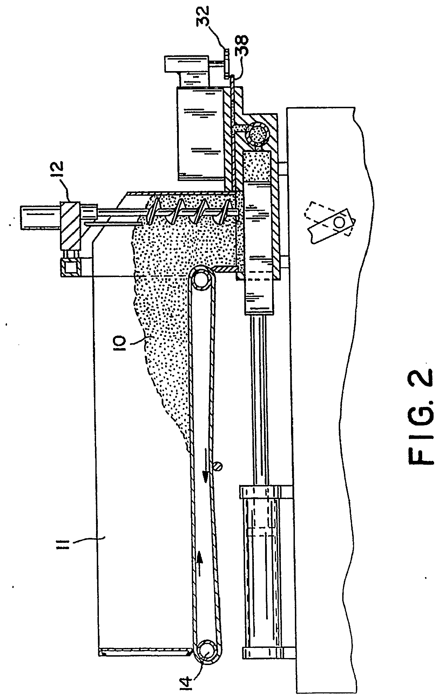

In one embodiment the forming step c) comprises the following steps referring to Figure 2:
a) Dough **10** is added to hopper **11;**
b) Screw(s) **12** and conveyer **14** move the dough **10** through feed screw(s) **30** and onto a mold plate **38;**
c) Knock out **32** then forms a soft chew.

### BRIEF DESCRIPTION OF THE DRAWINGS

Fig. 1a is an illustration of an embodiment of a composition of the present invention.
Fig. 1b is an illustration of an alternate embodiment of a composition of the present invention.
Fig. 2 is an illustration of an embodiment of a forming apparatus used in forming embodiments of compositions of the present invention.
Fig. 3 is an illustration of an embodiment of knock out used to form an embodiment of the present invention.

### Detailed Description of the Invention

As used herein, the term "cookie" and "soft chew" and any conjugation thereof, means and refers to an edible composition.

As used herein, the term "sugar," and any conjugation thereof, means and refers to any saccharide which is at least partially soluble in moisture, non-toxic, and preferably not provide any undesirable taste effects. Further, the use of the term "sugar" shall include a "sugar substitute."

As used herein, the term "sugar substitute," and any conjugation thereof, means and refers to any compound that produces a like effect as sugar, but does not require the same or magnitude of effect that a comparable amount of a sugar would produce.

As used herein, the term "Parasite," and any conjugation thereof, means and refers to a specie and/or species of organism treated by a pharmaceutical, non-limiting examples of which are included herein. Internal and external parasites of Equidae, Canidae, Felidae, Bovidae, Ovidae, Capridae, Suidae which include but are not limited to the pseudophyllidean and cyclophyllidean tapeworms, digenean flukes nematodes in the orders Rhabditida, Strongylida, Oxyurida, Ascaridida, Spirurida and Enoplida insects in the orders Siphonapter, Diptera, Mallophaga, Anpolura, and the suborders of arachnida including parasitic mites and ticks mesostigmata, astigmata, prostigmata and metastigmata. Exemplary, non-limiting parasites of horses (parasites of other species are also contemplated) include, but are not limited to, large strongyles, such as, but not limited to *Strongylus vulgaris, S. edentatus,* and *S*. *equines*; small strongyles including, but not limited to, those resistant to some benzimidazole class compounds, *Triodontophorus spp., Cyathostolnum spp., Cylicocyclusspp., Cylicostephanusspp.,* and *Cylicodontophorus spp.;* pinworms, such as, but not limited to *Oxyuris equi*; Ascarids, such as, but not limited to, *Parascaris equorum*; Hairworms, such as, but not limited to, *Trichostrongylus axei;* largemouth stomach worms, such as, but not limited to *Habronema muscae;* neck threadworms, such as, but not limited to *Onchocerca spp.;* bots, such as, but not limited to, *Gastrophilus spp.;* lungworms, such as, but not limited to, *Dictyocaulus arnfieldi;* intestinal threadworms, such as, but not limited to, *Strongyloides westeri;* summer sores caused by *Habronema* and *Draschia spp.,* and other cutaneous larvae; and other parasites that are common in the art. However, parasites of other species are specifically contemplated as falling within the scope of the invention.

As used herein, the term "amylaceous ingredients" is meant those food-stuffs containing a preponderance of starch and/or starch-like material. Examples of amylaceous ingredients are cereal grains and meals or flours obtained upon grinding cereal grains such as corn, oats, wheat, milo, barley, rice, and the various milling byproducts of these cereal grains such as wheat feed flour, wheat middlings, mixed feed, wheat shorts, wheat red dog, oat groats, hominy feed, and other such material. Also included as sources of amylaceous ingredients are the tuberous food stuffs such as potatoes, tapioca, and the like.

As used herein, percents of components of the soft chew means and refers to percentages of the total weight of the soft chew.

As used herein, the term "starch component" shall mean and refer to a starch or starches component and is considered a dry component, whether or not actually dry. As used herein, the term "sugar component" shall mean and refer to a sugar or sugars and/or sugar substitute component and is considered a dry component, whether or not actually dry. As used herein, the term "oil component" shall mean and refer to an oil or oils component and is considered a liquid component, whether or not actually liquid. As used herein, the term "additive component" shall mean and refer to an additive or additives. As used herein, the term "emulsifier component" means and refers to an emulsifier or emulsifiers, humectants and the like and is considered a liquid component, whether or not actually liquid.

Embodiments of a composition of the present invention are an edible delivery vehicle or soft chew for the delivery of an additive to an organism. Such organism may be any organism. Especially considered organisms include livestock, pets, farm animals, and the like, including, but not limited to, horses, cows, pigs, goats, sheep, llamas, deer, ducks, chickens, dogs, cats, lions, tigers, bears, oxen, buffalo, fish, birds, insects, and the like.

In embodiments according to the invention, the additive is a pharmaceutical. Embodiments of the soft chew of the present invention deliver reasonable levels of the additive, thereby producing the desired effect for the additive. The soft chew, in various embodiments, may be pleasant tasting and/or palatable to an organism.

In an embodiment, a composition of the present invention comprises a starch component, a sugar component, and an oil component. Generally, in various embodiments, the starch component comprises about 5 percent to about 60 percent of the soft chew, the sugar component comprises about 5 percent to about 75 percent of the soft chew, and the oil component comprises about 1 percent to about 40 percent of the soft chew. The percentages of the starch component, sugar component, and/or oil component may be varied depending upon the end use and desired consistency of the soft chew.

In an alternate embodiment, the starch component comprises about 15 percent to about 40 percent of the soft chew, the sugar component comprises about 15 percent to about 60 percent of the soft chew, and the oil component comprises about 5 percent to about 30 percent of the soft chew.

In an alternate embodiment, the starch component comprises about 25 percent to about 35 percent of the soft chew, the sugar component comprises about 25 percent to about 50 percent of the soft chew, and the oil component comprises about 7 percent to about 15 percent of the soft chew.

The starch component may comprise starch from any source and may act as a binder in the soft chew. In an embodiment, the starch component is derivatized and/or pregelatinized. In a preferred embodiment, the starch component is highly derivatized. Some starches that can serve as a base starch for derivatization include regular corn, waxy corn, potato, tapioca, rice, etc. Suitable types of derivatizing agents for the starch include, but are not limited to, ethylene oxide, propylene oxide, acetic anhydride, and succinic anhydride, and other food approved esters or ethers, introducing such chemicals alone or in combination with one another.

In various embodiments, prior cross-linking of the starch in the starch component may or may not be necessary, based on the pH of the system and the temperature used to form the product.

The starch component may also include amylaceous ingredients. The amylaceous ingredients can be gelatinized or cooked before or during the forming step to achieve the desired matrix characteristics. If gelatinized starch is used, it may be possible to prepare the product of the subject invention or perform the process of the subject invention without heating or cooking. However, ungelatinized (ungelled) or uncooked starch may also be used.

The sugar component may act as a sweetener and may comprise sugars including, but not limited to, white sugar, corn syrup, sorbitol (solution), maltitol (syrup), oligosaccharide, isomaltooligosaccharide, fructose, lactose, glucose, lycasin, xylitol, lactitol, erythritol, mannitol, isomaltose, polydextrose, raffinose, dextrin, galactose, sucrose, invert sugar, honey, molasses, polyhydric alcohols and other similar saccharides oligomers and polymers and mixture thereof. In addition, artificial sweeteners such as saccharine, aspartame and other dipeptide sweeteners may be present and sugarless can include solid polyols such as Sorbitol, Mannitol and Xylitol. Examples of various well established sources of a portion of these sugars are, corn syrup solids, malt syrup, hydrolyzed corn starch, hydrol (syrup from glucose manufacturing operations), raw and refined cane and beet sugars, and the like.

The oil component may act as a humectant and may comprise more than one oil including, but not limited to, fat or fats, both natural and synthetic. Oil employed as an ingredient in the soft chew may be a saturated or unsaturated liquid fatty acid, its glyceride derivatives or fatty acid derivatives of plant or animal origin or a mixture thereof. A source for typical animal fats or oils are fish oil, chicken fat, tallow, choice white grease, prime steam lard and mixtures thereof. However, other animal fats are also suitable for use in the soft chew. Suitable sources for vegetable fats or oils can be derived palm oil, palm hydrogenated oil, corn germ hydrogenated oil, castor hydrogenated oil, cotton-seed oil, soybean oil, olive oil, peanut oil, palm olein oil, Cacao fat, margarine, butter, shortening and palm stearin oil, and mixtures thereof. Additionally, a mixture of animal or vegetable oils or fats is suitable for use in the matrix.

Various other embodiments further comprise a flavoring component. Such flavoring component, in an embodiment, is to improve and/or change the palatability of the soft chew. Any flavoring in the flavoring component may be used. Examples of suitable flavor for the flavoring component includes, but is not limited to, strawberry flavor, tutti fruity flavor, orange flavor, banana flavor, mint flavor, and an apple-molasses. A suitable source for an apple-molasses flavoring component is Pharma Chemie, 1877 Midland Street, P.O. Box 326, Syracuse, NE 68446-0326, under a product name of Sweet-Apple Molasses Flavoring, Product Code PC-0555.

In various embodiments, other flavorings for the flavoring component may be used, such as fruit, meat (including, but not limited to pork, beef, chicken, fish, poultry, and the like), vegetable, cheese, cheese-bacon and/or artificial flavorings. In preferred embodiments utilizing a flavoring component, the flavoring component is chosen to enhance the palatability of the composition. A preferred meat flavoring is commercially available at Pharma Chemie as Artificial beef flavor product code PC-0125. A flavoring component is typically chosen based upon consideration related to the organism that will be ingesting the soft chew. For example, a horse may prefer an apple flavoring component, while a dog may prefer a meat flavoring component.

Various embodiments further comprise a stabilizer and/or lubricating component. In an embodiment, suitable stabilizer components are Magnesium Stearate, citric acid, sodium citrate, and/or the like. However, stabilizer components are common in the art and any suitable one or mixture of more than one may be used. In an embodiment, a stabilizer component comprises about 0.0 percent to about 3.0 percent of the soft chew. In an alternate embodiment, a stabilizer component comprises about 0.5 percent to about 1.5 percent of the soft chew.

Embodiments according to the invention further comprise an emulsifier component. A suitable emulsifier component is a glycerin, glycerin fatty acid ester, sorbitan monostearate, sucrose fatty acid ester, lecithin, polyethylene glycol, mixtures thereof, and the like.

According to the invention, the emulsifier component comprises polyethylene glycol as forming agent. However, emulsifier components are well-known in the art field and any emulsifier component may be used. Generally, the amount of emulsifier component added may affect the stickiness of the soft chew. The greater the concentration of glycerin, the stickier the soft chew. In an embodiment, an emulsifier component up to 40 percent of the soft chew. In an alternate embodiment, an emulsifier component comprises about 5.0 percent to about 30 percent of the soft chew. In an alternate embodiment, an emulsifier component comprises about 10 percent to about 20 percent of the soft chew.

In various embodiments, a moisture component is in the composition. In an embodiment, a moisture component comprises about 0.0 percent to about 15 percent. In an alternate embodiment, a moisture component comprises about 2.0 percent to about 10 percent. In an alternate embodiment, a moisture component comprises about 5.0 percent to about 7.5 percent.

In embodiments according to the invention, an additive component is added to the composition. The additve component is a pharmaceutical, that can be orally administered. In this regard, an additive component may be an active ingredient or an inactive ingredient.

Exemplary pharmaceuticals include, but are not limited to, ivermectin, fenbendazole, piperazine, magnesium hydroxide, stranozole, furosemide, penicillin, amoxicillin, prednisolone, methylprednisolone, acepromazine, aspirin, PROZAC, ZANTACS, BENADRYL, praziquantel, pyrantel, HOE 12073, Sumitomo Chemicals-1638, Nitenpyram, spinosad and omyprazole.

Various embodiments of the present invention contemplate a soft chew with more than one pharmaceutical/additive. Exemplary embodiments with more than one pharmaceutical include, without limitation, ivermectin with Omeprazole, with Fenbendazole, with Pyrantel pamoate to be used in rotation with fenbendazole, with fenbendazole and praziquantel, with ivermectin and pyrantel pamoate, with Selamectin, with Praziquantel and pyrantel pamoate for cats, milbemycin and praziquantel for dogs and cats to control nematodes and tapeworms, furosemide in dogs, cats, horses, cattle. Further embodiments substitutes praziquantel for epsiprantel, fenbendazole for benzimidazole, ivermectin for macrolide anthelmintic, and the like. Generally, any drug that is given as a tablet could be put in this soft chew, as long as the excipients in the formulation wouldn't cause a stability problem or combine with them is such a way as to make them inactive. Specifically contemplated soft chews of the present invention may be identified as single additive, dual additive, three-way, four-way, and so on. In such embodiments, the soft chews comprise a first additive and/or a second additive and/or a third additive and/or a fourth additive and/or so on. Accordingly, an additive component of the present invention may comprise more than one additive. Preferably, additives are chosen that will not interfere with one another, so as not to make a non-operative soft chew.

In various embodiments, the additive is coated. Any suitable coating may be used. In an embodiment, a coating is chosen that will not interfere with an additive. In another embodiment, an additive is chosen that can modify the time for digestion of the additive(s), thereby at least partially controlling the release of the additive(s). Suitable coatings include, but are not limited to, and may be any pharmaceutically acceptable, and/or neutraceutically acceptable coating, as is common in the art.

Exemplary fillers include, but are not limited to, a carbohydrate source, a protein source, antioxidants, such as Tenox 8, gum, colorants, dyes, pigments, and the like. Generally, any ingredient may be used as a filler. In preferred embodiments, the filler is chosen so as not to adversely affect the palatability of the soft chew.

An embodiment of a process for forming a soft chew of the present invention comprises the steps of:
mixing a starch component, a sugar component, an oil component, and an additive component;
optionally heating at least a portion of the components; and,
forming embodiments of the soft chew.

If an additive is present in the embodiment of the soft chew, the additive component may be mixed along with the other components or at a later step and/or time in the process. In an alternate embodiment, the components are mixed completely to produce a mixed dough. In a most preferred embodiment, the dough is mixed until there is a uniform dispersal of the components in the dough.

In a further embodiment, the process further comprises mixing an emulsifier component. The emulsifier component may be chosen to act as a humectant and/or a forming agent. In an embodiment, a forming agent of choice is polyethylene glycol (PEG). Moreover, depending upon the desired consistency of the soft chew, different molecular weight PEG may be utilized. In an embodiment, PEG 3350 is utilized. However, the PEG chosen is a matter of choice and the molecular weight may be higher or lower than 3350.

Embodiments of processes of the present invention may further comprise mixing a stabilizer component, a flavoring component, and/or a filler component.

In an embodiment, the dry components are mixed and the liquid components are mixed separately. In an embodiment, the oil component and the emulsifier component are heated when mixing and added, at sufficient temperature, to the dry components. The liquid and dry components are then mixed together until a desired dough is obtained. However, the process by which the components are mixed and/or heated into a dough may be varied. Moreover, the degree of mixing may be varied, such that, in various embodiments, the dough is not uniformly mixed and remains striated. Likewise, various embodiments of dough of the present invention have discrete zones and/or layers.

In an embodiment, an additive(s) component is added during mixing of the components. In an alternate embodiment, an additive component is injected into the soft chew after forming. In an alternate embodiment, a dough is formed about an additive component. In another embodiment, an additive(s) is mixed and/or dissolved in an alcohol or other liquid prior to adding with a dough and/or components of the present invention. In alternate embodiments, an additive(s) component is sprayed into a dough while mixing. The particular process for mixing the additive in the dough may be dependant upon considerations, including the stability of the additive, the temperature sensitivity of the additive, and/or the like. In a preferred embodiment, the additive is uniformly mixed and/or dispersed in the dough.

In another embodiment, the oil component is heated prior to mixing the components,

The dough is then formed into a soft chew of the present invention by a knockout. In an embodiment, the dough is formed while still warm. The dough may be formed into a soft chew by any means or method common in the art, such as by hand or by machine. In an embodiment, a forming machine or patty machine is utilized, such that the soft chew is formed out of the dough. Suitable examples of forming machines are exemplified in U.S. Pat. Nos. 5,165,218, 7,780,931, 4,523,520, and 3,887,964. A most preferred forming machine is the FORMAX machine manufactured by FORMAX Food Machines, Mokena, Illinois.

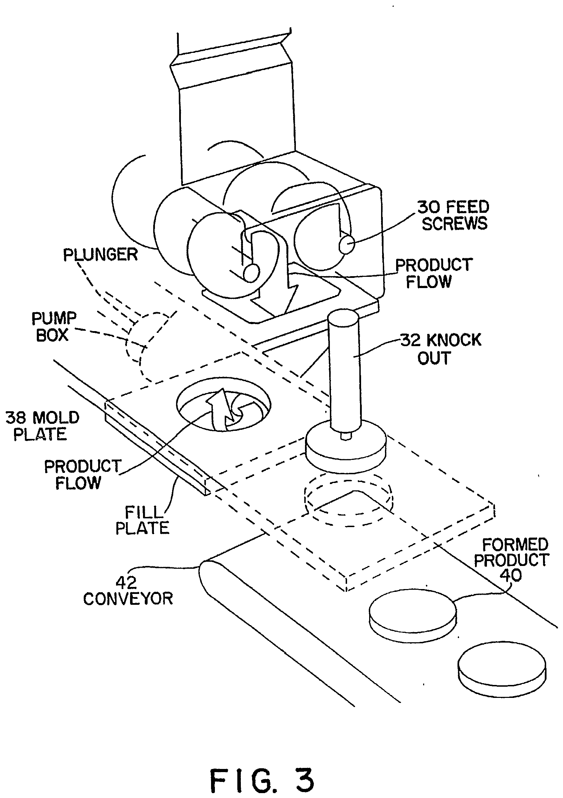

Now referring to Figure 2, an illustration of an embodiment of a forming apparatus used in forming embodiments of compositions of the present invention, a general preferred embodiment for forming soft chews of the present invention will be discussed. Generally, dough 10 is added to hopper 11. Screw(s) 12 and conveyor 14 move dough 10 through feed screw(s) 30 and onto mold plate 38. Knock out 32 then forms a soft chew of the present invention. Reference to Figure 3, an illustration of an embodiment of knock out used to form an embodiment of the present invention, illustrates a preferred embodiment of soft chew 40. However, any size or shape knock out is acceptable. Soft chew 40 is then conveyed along conveyor 42. Soft chew 40 may be used, packaged, or as is desired.

In an embodiment, dough 10 is formed into soft chew 40 while still warm. However, dough 10 may be formed into soft chew 40 at any desired temperature.

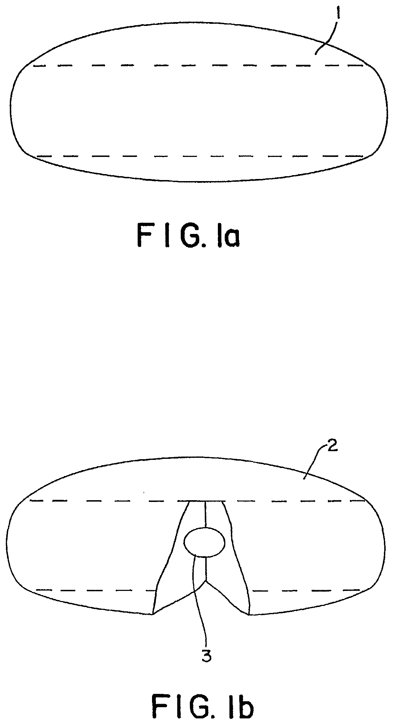

Embodiments of a soft chew of the present invention may have different textures, crispyness, hardness, and the like. In an embodiment texture of the soft chew will be smooth. In other embodiments, the texture of the soft chew will be rough. Now referring to Figure 1a, an embodiment of a soft chew of the present invention, a soft chew 1 that was formed from a uniformly mixed dough is illustrated. However, Figure 1b illustrates a soft chew 2 with more than one zone. Whether the dough is uniformly mixed or not may be dependent upon various factors, including the type of additive, consistency of soft chew, and/or the like.

Further embodiments of the present invention are for a process of introducing an additive to an organism. Suitable examples of organisms are livestock, pets, farm animals, and the like, including, but not limited to, horses, cows, pigs, goats, sheep, llamas, deer, ducks, chickens, dogs, cats, lions, tigers, bears, oxen, buffalo, fish, birds, insects, and the like. In an embodiment, the organism is a horse and the additive is ivermectin and/or other pharmaceutical and the process of treating a horse for worms comprises the steps of: obtaining an embodiment of a soft chew of the present invention and offering the soft chew to the horse whereby, upon consumption, ivermectin and/or other pharmaceutical(s) is released.

Dosage requirements for embodiments of the present invention will vary and should be chosen to be within established veterinary parameters. In various embodiments, dosage delivered to an organism may be adjusted by offering more or less units of soft chews to the organism, one soft chew supplies enough additive for a certain weight organism.

While the invention has been described in connection with specific embodiments thereof, it will be understood that it is capable of further modifications and the appended Claims are intended to cover any variations, uses, or adaptations of the invention following, in general, the principles of the invention and including such departures from the present disclosure as come within known or customary practice within the art to which the invention pertains and as may be applied to the essential features hereinbefore set forth whether now existing or after arising. Further, while embodiments of the invention have been described with specific dimensional characteristics and/or measurements, it will be understood that the embodiments are capable of different dimensional characteristics and/or measurements without departing from the principles of the invention and the appended Claims are intended to cover such differences.

For a further understanding of an embodiment of the present invention, reference should be had to the following examples:

### Examples:

The following placebo experiments were conducted to test the palatability of embodiments of a soft chew of the present invention:

### Example 1a: Equine Soft Chew

### Mixture of Components:

A batch of placebo dough was made with the following components: Each component is illustrated in grams/batch. There was no additive added.

| Ingredients | gram/batch |
|---|---|
| Sweet Apple'& Molasses Flavor (PC-0555) | 2699.3 |
| Corn Starch, NF | 2800.0 |
| Sucrose, NF | 1500.0 |
| Magnesium Stearate, NF | 100.0 |
| Soybean Oil, USP | 700.0 |
| Glycerin, USP | 1300.0 |
| Tenox 8 | 0.7 |
| Polyethylene Glycol, NF 3350 | 900.0 |
| Total Lot Weight: | 10000.0 g |

The process used for mixing the components was as follows:
1. All the components were weighed.
2. All the dry components, beginning with the Sweet Apple & Molasses Flavor , then the corn starch, then the sucrose, and then the magnesium stearate were mixed in a 20 quart (18.921) Hobart mixer at speed 1 for about 1 minute, to produce a uniform blend.
3. The liquid components, beginning with the soybean oil, then the glycerin, and then the tenox 8 were mixed in a steel beaker with a spatula.
4. The polyethylene glycol was then heated to 60° C in a stainless steel beaker to form a liquid.
5. The liquid from step 3 was then added to the Hobart mixer and mixed for 5 minutes to produce a wet granulation mix.
6. The melted PEG from step 4 was then added to the Hobart mixer and mixed for 15 minutes to produce a wet granulation mix.
7. The wet granulation mix was then added to a Formax F6 machine to produce soft chews of the present invention.

### Physical Characteristics of the Soft chew:

The soft chews formed from the above section had the following general characteristics:

| | |
|---|---|
| Soft chew Weight: | 4850.0 mg |
| Soft chew Hardness | 0.0 Kp (N) |
| Soft chew Thickness | 0.521 inches (13.23 mm) |
| Soft chew Friability | 0.00 % |
| Time to Disintegrate | 14.45 minutes |
| (6 soft chews, 37 °C) | |

### Control Soft chew:

A control soft chew was chosen for comparison of the palatability of the placebo embodiment of the present invention. A product identified as MRS. PASTURES' SOFT CHEWS FOR HORSES was chosen as a control. The control contained oats, wheat, bran, cane molasses, rolled barley, apples and water. The control soft chew weighed 10 grams.

### Palatability Study:

Apalatability study was conducted as follows:
1. Twenty horses were evaluated over a two day period with the placebo as compared to the control with two offerings in a day.
2. Two product offerings were made to each horse on each day. One offering was four of the placebo soft chews and the other offering was one of the control soft chews. Each product offering was divided into two parts. The first part was a free choice evaluation where each test horse was offered, for consumption, either the control or an embodiment of the present invention. If the test horse refused the soft chew on free choice, the soft chew was placed in the test horse's feed to see if the horse would consume the soft chew.
3. The study was repeated on a subsequent day in a varied order of offerings to remove experimental bias.
100.0% of the test horses consumed the control upon free choice. 97.5% of the test horses consumed the soft chew of the present invention upon free choice. 2.5% of the test horses consumed the soft chew of the present invention upon placing the soft chew in the horse's feed. Therefore, all of the test horses consumed the soft chew. The results of the study illustrate that an embodiment of a soft chew of the present invention is palatable for a horse.

### Example 1b: Equine Soft Chew with Ivermectin

### Mixture of Components:

A batch of dough with active/additive ingredient was made with the following components: Each component is illustrated in grams/batch.

| Ingredients | gram/batch |
|---|---|
| Ivermectin (97.6% purity)(5% overage) | 101.979 |
| Sweet Apple & Molasses Flavor (PC-0555) | 2499.3 |
| Corn Starch, NF | 500.0 |
| Sucrose, NF | 3898.021 |
| Magnesium Stearate, NF | 100.0 |
| Soybean Oil, USP | 700.0 |
| Glycerin, USP | 1300.0 |
| Tenox 8 | 0.7 |
| Polyethylene Glycol, NF 3350 | 900.0 |
| | |
| Total Lot Weight: | 10000.0 g |

The process used for mixing the components was as follows:
1. All the components were weighed.
2. All the dry components, beginning with the Sweet Apple & Molasses Flavor , then the corn starch, then the sucrose, and then the magnesium stearate were mixed in a 20 quart (18.92 1) Hobart mixer at speed 1 for about 1 minute, to produce a uniform blend.
3. The liquid components, beginning with the soybean oil, then the glycerin, and then the tenox 8 were mixed in a steel beaker with a spatula.
4. The polyethylene glycol was then heated to 60° C in a stainless steel beaker to form a liquid.
5. The liquid from step 3 was then added to the Hobart mixer and mixed for 5 minutes to produce a wet granulation mix.
6. The melted PEG from step 4 was then added to the Hobart mixer and mixed for 15 minutes to produce a wet granulation mix.
7. The wet granulation mix was then added to a Fonnax F6 machine to produce soft chews of the present invention.

### Physical Characteristics of the Soft chew:

The soft chews formed from the above section had the following general characteristics:

| | |
|---|---|
| Soft chew Weight: | 4838.7 mg |
| Soft chew Hardness | 0.0 Kp (N) |
| Soft chew Thickness | 0.504 inches (12.8 mm) |
| Soft chew Friability | 0.08 % |
| Time to Disintegrate | 14.45 minutes |
| (6 soft chews, 37 °C) | |

### Palatability Study:

100.0% of the test horses consumed the control upon free choice. 95.0% of the test horses consumed the soft chew of the present invention upon free choice. 5.0% of the test horses consumed the soft chew of the present invention upon placing the soft chew in the horse's feed. Therefore, all of the test horses consumed the soft chew. The results of the study illustrate that an embodiment of a soft chew of the present invention, with additive, is palatable for a horse.

### Example 2: Canine Soft Chew

### Mixture of Components:

A batch of dough with active/additive ingredients was made with the following components: Each component is illustrated in grams/batch. There were no additive(s) added.

| Ingredients | gram/batch |
|---|---|
| Artificial Beef Flavor (PC-0125) | 810.0 |
| Sweet Apple & Molasses Flavor (PC-0555) | 594.0 |
| Corn Starch, NF | 79.567 |
| Fenbendazole Granulation | 1890.0 |
| Praziquantel | 59.051 |
| Aspartame, NF | 59.40 |
| Ivermectin Concentrate (5% overage) | 71.442 |
| Magnesium Stearate, NF | 54.0 |
| Soybean Oil, USP | 594.0 |
| Glycerin, USP | 702.0 |
| Tenox 8 | 0.54 |
| Polyethylene Glycol, NF 3350 | 486.0 |
| | |
| Total Lot Weight: | 5400.0 g |

The process used for mixing the components was as follows:
1. All the components were weighed.
2. All the dry components, beginning with the Sweet Apple & Molasses Flavor, the Artificial Beef Flavor, then the corn starch, the active ingrediemts, then the sucrose, and then the magnesium stearate were mixed in a20 quart (18.92 1) Hobart mixer at speed 1 for about 1 minute, to produce a uniform blend.
3. The liquid components, beginning with the soybean oil, then the glycerin, and then the tenox 8 were mixed in a steel beaker with a spatula.
4. The polyethylene glycol was then heated to 70° C in a stainless steel beaker to form a liquid.
5. The liquid from step 3 was then added to the Hobart mixer and mixed for 5 minutes to produce a wet granulation mix.
6. The melted PEG from step 4 was then added to the Hobart mixer and mixed for 10 minutes to produce a wet granulation mix.
7. The wet granulation mix was then added to a Formax F6 machine to produce soft chews of the present invention.

### Physical Characteristics of the Soft chew:

The soft chews formed from the above section had the following general characteristics:

| | |
|---|---|
| Soft chew Weight: | 5292.0 mg |
| Soft chew Hardness | 0.0^{∗} Kp (N) |
| Soft chew Thickness | 0.588 inches (14.94 mm) |
| Soft chew Friability | 0.18 % |
| Time to Disintegrate | >60.0 minutes |
| (6 soft chews, 37 °C) | |

| | |
|---|---|
| *Soft Chew was to soft, no reading | |

### Control Soft chew:

A control soft chew was chosen for comparison of the palatability of this embodiment of the present invention. A product identified as the Interceptor 23 mg Flavor Tablet, prepared by Novartis Animal Health Co., was chosen as a control. The control was a direct compression product, 960 mg/tablet.

### Palatability Study:

A palatability study was conducted as follows:
1. Twenty (20) canines were randomly selected. The breeds selected included Labradors, Shelties, Collies, Rottweilers, and other mixed breeds. The weight of the canines was between 20 pounds and 150 pounds. The age range was 1 year to 14 years, with a mean age of 4.6 years. The canines were evenly divided between males and bitches.
2. Each canine was presented with two product offers per day over a two day period. One product offer of a soft chew of the present invention and one product offer of the control, Interceptor product. This resulted in 40 tests.
3. Statistics were taken for % palatability (free choice), % palatable (with food), and % refused. The soft chews results indicated that 85.0% of the canines took the soft chew of the present invention (free choice), 7.5% of the canines took the soft chew of the present invention (with food), therefore, leaving only a 7.5% refusal. The control Interceptor product's results indicated that 75.8% of the canines took the soft chew of the present invention (free choice), 21.7% of the canines took the soft chew of the present invention (with food), therefore, leaving only a 2.5% refusal. As can be seen, the embodiment of the soft chew of the present invention performed better than the control, was accepted at a greater percentage through free choice.

## Claims

1. A soft chew, the soft chew comprising:
a flavoring component of between 0.1 to 50 percent wt,
a starch component of between 5 to 60 percent wt,
a sugar component, between 5.0 to 75 percent wt,
an oil component between 1.0 to 40 percent wt;
and a pharmaceutical as a first additive,
wherein the moisture content is less than 15 percent wt;
wherein the soft chew is formed by knockout, the soft chew is not an extrudate;
wherein the composition further comprises an emulsifier component comprising polyethylene glycol as forming agent.

2. The soft chew according to claim 1, wherein the soft chew comprises between 5 to 30 percent wt of the emulsifier component.

3. The soft chew according to claim 1 or 2, wherein the sugar component act as a sweetener and is selected from white sugar, corn syrup, sorbitol solution, maltitol syrup, oligosaccharide, isomaltooligosacccharide, fructose, lactose, glucose, lycasin, xylitol, lactitol, erythritol, mannitol, isomaltose, polydextrose, raffinose, dextrin, galactose, sucrose, invert sugar, honey, molasses, polyhydric alcohol and mixtures thereof.

4. The soft chew according to any one of claims 1 to 3, wherein the source of the sugar component are corn syrup solids, malt syrup, hydrolyzed corn starch, hydrol, or raw and refined cane or beet sugars.

5. The soft chew of any one of claims 1 to 4 wherein the soft chew comprises more than one pharmaceutical.

6. The soft chew according to any one of claims 1 to 5 comprising polyethylene glycol of different molecular weight.

7. The soft chew according to any one of claims 1 to 6 comprising PEG 3350.

8. The soft chew according to any one of claims 1 to 7 comprising a meat flavoring component.

9. A process for forming a soft chew comprising the steps of:
a) mixing a starch component, a sugar component, an oil component and an emulsifier component comprising polyethylene glycol and a pharmaceutical as first additive component to produce a dough;
b) heating at least a portion of the components;
c) Forming the dough into a soft chew in a forming machine with a knockout, such that the soft chew is not an extrudate.

10. The process according to claim 9 wherein the oil component and the emulsifier component are heated when mixing.

11. The process according to claims 9 or 10 wherein the emulsifier component comprises polyethylene glycol of different molecular weight.

12. The process according to any one of claims 9 to 11 wherein the emulsifier component comprises PEG 3350.

13. The process according to any one of claims 9 to 12 wherein in step a) the pharmaceutical as first additive is mixed and/ or dissolved in an alcohol or other liquid prior to adding with a dough and/or components of the soft chew.

14. The process according to any one of claims 9 to 13 wherein a soft chew as described in claims 1 to 8 is formed.

15. The process according to any one of claims 9 to 14, wherein the step of knocking out is performed on a patty pressing machine.

16. The process according to any one of claims 9 to 15 wherein the forming step c) comprises the following steps:
a. Dough **10** is added to hopper **11;**
b. Screw(s) **12** and conveyer **14** move the dough **10** through feed screw(s) **30** and onto a mold plate **38;**
c. Knock out **32** then forms a soft chew.

## Patentansprüche

1. Weiches Kauprodukt, wobei das weiche Kauprodukt umfasst:
eine Aromakomponente von zwischen 0,1 und 50 Gew.-%,
eine Stärkekomponente von zwischen 5 und 60 Gew.-%,
eine Zuckerkomponente, zwischen 5,0 und 75 Gew.-%,
eine Ölkomponente, zwischen 1,0 und 40 Gew.-%,
und ein Pharmazeutikum als einen ersten Zusatzstoff,
wobei der Feuchtegehalt weniger als 15 Gew.-% beträgt;
wobei das weiche Kauprodukt durch Auswerfen gebildet ist, wobei das weiche Kauprodukt kein Extrudat ist;
wobei die Zusammensetzung ferner eine Emulgatorkomponente umfasst, die Polyethylenglycol als Formmittel umfasst.

2. Weiches Kauprodukt gemäß Anspruch 1, wobei das weiche Kauprodukt zwischen 5 und 30 Gew.-% der Emulgatorkomponente umfasst.

3. Weiches Kauprodukt gemäß Anspruch 1 oder 2, wobei die Zuckerkomponente als Süßstoff wirkt und ausgewählt ist aus weißem Zucker, Maissirup, Sorbitlösung, Maltitsirup, Oligosaccharid, Isomaltooligosaccharid, Fructose, Lactose, Glucose, Lycasin, Xylit, Lactit, Erythrit, Mannit, Isomaltose, Polydextrose, Raffinose, Dextrin, Galactose, Saccharose, Invertzucker, Honig, Molassen, polyhydrischem Alkohol und Gemischen davon.

4. Weiches Kauprodukt gemäß einem der Ansprüche 1 bis 3, wobei die Quelle der Zuckerkomponente Maissirup-Feststoffe, Malzsirup, hydrolysierte Maisstärke, Hydrol oder roher und raffinierter Rohr- oder Rübenzucker ist.

5. Weiches Kauprodukt gemäß einem der Ansprüche 1 bis 4, wobei das weiche Kauprodukt mehr als ein Pharmazeutikum umfasst.

6. Weiches Kauprodukt gemäß einem der Ansprüche 1 bis 5, umfassend Polyethylenglycol mit verschiedenem Molekulargewicht.

7. Weiches Kauprodukt gemäß einem der Ansprüche 1 bis 6, umfassend PEG 3350.

8. Weiches Kauprodukt gemäß einem der Ansprüche 1 bis 7, umfassend eine Fleischaromakomponente.

9. Verfahren zur Herstellung eines weichen Kauprodukts, umfassend die Schritte:
a) Mischen einer Stärkekomponente, einer Zuckerkomponente, einer Ölkomponente und einer Emulgatorkomponente, die Polyethylenglycol umfasst, und eines Pharmazeutikums als erste Zusatzstoffkomponente, um einen Teig zu bilden;
b) Erhitzen wenigstens eines Teils der Komponenten;
c) Formen des Teigs zu einem weichen Kauprodukt in einer Formvorrichtung mit einem Auswerfer, so dass das weiche Kauprodukt kein Extrudat ist.

10. Verfahren gemäß Anspruch 9, wobei die Ölkomponente und die Emulgatorkomponente bei dem Mischen erhitzt werden.

11. Verfahren gemäß Ansprüchen 9 oder 10, wobei die Emulgatorkomponente Polyethylenglycol mit verschiedenem Molekulargewicht umfasst.

12. Verfahren gemäß einem der Ansprüche 9 bis 11, wobei die Emulgatorkomponente PEG 3350 umfasst.

13. Verfahren gemäß einem der Ansprüche 9 bis 12, wobei bei Schritt a) das Pharmazeutikum als erster Zusatzstoff vor dem Zugeben zu einem Teig und/oder Komponenten des weichen Kauprodukts mit/in einem Alkohol oder einer anderen Flüssigkeit gemischt und/oder gelöst wird.

14. Verfahren gemäß einem der Ansprüche 9 bis 13, wobei ein weiches Kauprodukts gemäß Ansprüchen 1 bis 8 gebildet wird.

15. Verfahren gemäß einem der Ansprüche 9 bis 14, wobei der Schritt des Auswerfens auf einer Bratlingpressmaschine durchgeführt wird.

16. Verfahren gemäß einem der Ansprüche 9 bis 15, wobei der Formschritt c) folgende Schritte umfasst:
a. ein Teig **10** wird zu einem Fülltrichter **11** zugegeben;
b. eine Schnecke/Schnecken **12** und eine Fördereinrichtung **14** bewegt/bewegen den Teig **10** durch eine Förderschnecke/Förderschnecken **30** und auf eine Formplatte **38;**
c. anschließend bildet ein Auswerfer **32** ein weiches Kauprodukt.

## Revendications

1. Article croquable mou, l'article croquable mou comprenant :
un composant d'arôme allant de 0,1 à 50% en poids,
un composant d'amidon allant de 5 à 60% en poids,
un composant de sucre allant de 5,0 à 75% en poids,
un composant d'huile allant de 1,0 à 40% en poids,
et une substance pharmaceutique comme premier additif,
où la teneur en humidité est inférieure à 15% en poids ; où l'article croquable mou est formé par poinçonnage, l'article croquable mou n'étant pas un extrudat ;
où la composition comprend en outre un composant d'émulsifiant comprenant du polyéthylène glycol comme agent de formage.

2. Article croquable mou selon la revendication 1, l'article croquable mou comprenant de 5 à 30% en poids du composant d'émulsifiant.

3. Article croquable mou selon la revendication 1 ou 2, où le composant de sucre joue un rôle d'édulcorant et est choisi parmi le sucre blanc, le sirop de glucose, une solution de sorbitol, un sirop de maltitol, un oligosaccharide, un isomalto-oligosaccharide, le fructose, le lactose, le glucose, la lycasine, le xylitol, le lactitol, l'érythritol, le mannitol, l'isomaltose, le polydextrose, le raffinose, une dextrine, le galactose, le saccharose, le sucre inverti, le miel, les mélasses, l'alcool polyhydrique, et des mélanges de ceux-ci.

4. Article croquable mou selon l'une quelconque des revendications 1 à 3, où la source du composant de sucre est constituée par le sirop de glucose déshydraté, le sirop de malt, l'amidon de maïs hydrolysé, l'hydrol, ou des sucres de betterave ou de canne à sucre bruts et raffinés.

5. Article croquable mou selon l'une quelconque des revendications 1 à 4, l'article croquable mou comprenant plus d'une substance pharmaceutique.

6. Article croquable mou selon l'une quelconque des revendications 1 à 5, comprenant des polyéthylène glycols de poids moléculaire différent.

7. Article croquable mou selon l'une quelconque des revendications 1 à 6, comprenant du PEG 3350.

8. Article croquable mou selon l'une quelconque des revendications 1 à 7, comprenant un composant d'arôme de viande.

9. Procédé de formation d'un article croquable mou, comprenant les étapes consistant à :
a) malaxer un composant d'amidon, un composant de sucre, un composant d'huile et un composant d'émulsifiant comprenant du polyéthylène glycol et une substance pharmaceutique comme premier additif, afin de produire une pâte ;
b) chauffer au moins une portion des composants ;
c) façonner la pâte en un article croquable mou dans une façonneuse avec un poinçonnage, de façon à ce que l'article croquable mou ne soit pas un extrudat.

10. Procédé selon la revendication 9, dans lequel le composant d'huile et le composant d'émulsifiant sont chauffés lors du malaxage.

11. Procédé selon les revendications 9 ou 10, dans lequel le composant d'émulsifiant comprend des polyéthylène glycols de poids moléculaire différent.

12. Procédé selon l'une quelconque des revendications 9 à 11, dans lequel le composant d'émulsifiant comprend du PEG 3350.

13. Procédé selon l'une quelconque des revendications 9 à 12, dans lequel, dans l'étape a), la substance pharmaceutique comme premier additif est mélangée et/ou solubilisée dans un alcool ou un autre liquide préalablement à l'addition à une pâte et/ou des composants de l'article croquable mou.

14. Procédé selon l'une quelconque des revendications 9 à 13, dans lequel un article croquable mou tel que décrit selon les revendications 1 à 8, est formé.

15. Procédé selon l'une quelconque des revendications 9 à 14, dans lequel l'étape de poinçonnage est mise en œuvre dans une machine à presser des articles de type steak haché.

16. Procédé selon l'une quelconque des revendications 9 à 15, dans lequel l'étape de façonnage c) comprend les étapes suivantes :
a. de la pâte **10** est ajoutée à la trémie **11** ;
b. une ou plusieurs vis **12** et une bande transporteuse **14** déplacent la pâte **10** via la ou les vis d'alimentation **30** et dans une plaque de moulage **38** ;
c. le poinçonnage **32** façonne ensuite un article croquable mou.
